# EUROPEAN PATENT APPLICATION

(11) **EP 2 436 758 A2**
(43) Date of publication of application: **04.04.2012**
(21) Application number: 10780789.3
(22) Date of filing: 26.05.2010
(51) Int. Cl.: C12N 5/04, C12N 5/02

(54) **PLANT STEM CELL DERIVED FROM CAMBIUM OF FAMILY GINGKOACEAE AND METHOD FOR THE ISOLATION THEREOF**

(30) Priority: 26.05.2009 KR 20090045954
(71) Applicant: Unhwa Corporation, Jeollabuk-do 561-211 (KR)
(72) Inventor: HONG, Nam Ju, Gangjin-gun Jeollanam-do 527-821 (KR); JIN, Young Woo, Jeonju-si Jellabuk-do 561-808 (KR); LEE, Eun Kyong, Iksan-si Jeollabuk-do 570-976 (KR); HONG, Sun Mi, Jeonju-si Jeollabuk-do 561-231 (KR)
(74) Representative: Kiddle, Simon John
(86) International application number: PCT/KR2010/003341
(87) International publication number: WO 2010/137878

(57) **Abstract**

The present invention relates to a stem cell derived from a cambium of family *Ginkgoaceae* and a method of isolating the same. The stem cell derived from the cambium of the family *Ginkgoaceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell line has an antioxidant effect which is similar to or higher than that of an existing antioxidant, as demonstrated by measuring the ability to eliminate radicals caused by oxidants. Thus, the disclosed stem cell line is useful as an effective antioxidant composition.

## Description

### TECHNICAL FIELD

The present invention relates to a stem cell derived from a cambium of family *Ginkgoaceae* and a method of isolating the same.

### BACKGROUND ART

Plants were recognized as food resources in the past, but the meaning thereof is currently expanded to include a source for a wide range of chemical substances, including drugs, perfumes, pigments, agricultural chemicals and dyes and etc. Particularly, because most useful substances derived from plants have physiological activities, including antiviral, antibacterial, anticancer and antioxidant activities, plants are considered as ideal resources which can be developed into novel drugs, and active studies are in progress to elucidate the relationship between the chemical structures and activities of many plant-derived substances.

However, physiologically active substances are difficult to develop into drugs, and the main reasons therefore are as follows. First, the contents of physiologically active substances in plants are very limited. Second, the growth rate of plants is very slow. Third, physiologically active substances derived from plants are present only in specific organs of plants in small amounts. Fourth, environmental problems associated with the destruction of nature are involved. Fifth, physiologically active substances derived from plants have very complicated chemical structures, such that multi-step polymerization processes are required, thus causing economic problems of high production costs. For these reasons, it was very difficult to stably supply physiologically active substances derived from plants for commercialization.

Meanwhile, one of bioengineering techniques, that is a plant cell culture method, has been evaluated for a long time as the most ideal technique which can supply plant-derived useful substances without causing environmental problems. According to Korean Patent Publication No. 1995-0000870 (January 3, 1995), the production of useful substances by plant cell culture technique provides many advantages over methods of extracting useful substances directly from plants. Particularly, the plant cell culture method has been regarded as an optimal method which allows continuous production without being influenced by external environments so as to solve outstanding problems such as ecosystem destruction, unlike the prior extraction methods. However, despite great interest and effort in plant cell culture, examples which succeeded in industrializing the plant cell culture are still insufficient. This is because the variations in cell growth and productivity in a number of plant cell cultures still remain as a major problem.

In a case where plant cells are used in plant expression systems, a de-differentiation process is necessarily carried out in advance in order to convert tissues into a cell line having the ability to divide because the tissues which differentiated from the plant cells, for example, leaves, stems and seeds, are permanent tissues whose cells no longer divide. The de-differentiation process means de-differentiating tissues or cells, which have already been differentiated so as to perform specific functions, when a plant tissue or organ is cultured. However, during this de-differentiation process, serious changes in the cell line can occur due to chromosomal variations.

Particularly, the production of useful substances through plant cell culture can be industrialized, only when rapid cell growth and high metabolite productivity are stably maintained during a long-term culture period. However, most cells undergo many variations different from the original due to subculture. Thus, it is urgent to overcome this problem of variations in cells and to develop a method for acquiring a genetically stable cell line in producing useful substances through plant cell culture.

Korean Patent Registration 10-0533120 developed by some of the present inventors discloses a method of inducing callus using the cambium collected from the stem of a plant. However, in this registered patent, the callus is merely induced from the cambium of woody plant stem. This registered patent still has the problem of variation caused by dedifferentiation because the callus is a tissue formed through a dedifferentiation process.

Furthermore, some of the present inventors developed the invention of PCT/KR 2006/001544, which solves the problem of variation caused by dedifferentiation and relates to a method for providing cell lines that can stably proliferate and have high genetic stability. However, a ginkgo tree that is a deciduous tree is widely known as a useful plant, and in the case of this ginkgo tree, there has been a need to obtain a cell line that overcomes the problem of variation caused by dedifferentiation, can stably proliferate and has high genetic stability.

Accordingly, the present inventors have made extensive efforts to obtain a useful plant cell line by isolating a stem cell derived from a cambium of family *Ginkgoaceae.* As a result, the present inventors have isolated a stem cell derived from the cambium of the family *Ginkgoaceae* and have found that the isolated stem cell stably proliferates and does not vary during culture, thereby completing the present invention.

### DISCLOSURE OF INVENTION

Therefore, it is an object of the present invention to provide a stem cell derived from a cambium of family *Ginkgoaceae,* in which the stem cell has not undergone a dedifferentiation process, and a method of isolating the stem cell.

To achieve the above object, the present invention provides a stem cell derived from a cambium of family *Ginkgoaceae,* in which the stem cell is an innately undifferentiated cell without going through dedifferentiation.

The present invention also provides a method of isolating a stem cell derived from a cambium of family *Ginkgoaceae,* the method comprising the steps of:
(a) obtaining a tissue comprising a cambium from a plant of family *Ginkgoaceae*;
(b) culturing the obtained cambium-comprising tissue in a medium; and
(c) isolating a cell from the cambium, thereby obtaining a stem cell derived from the cambium.

The present invention also provides an antioxidant composition containing any one or more of said stem cell derived from the cambium of the family *Ginkgoaceae,* an extract thereof, a lysate thereof, and a culture thereof.

The present invention also provides an antioxidant functional food containing any one or more of said stem cell derived from the cambium of the family *Ginkgoaceae,* an extract thereof, a lysate thereof, and a culture thereof.

Other features and embodiments of the present invention will be more apparent from the following detailed descriptions and the appended claims

### BRIEF DESCRIPTION OF THE DRAWINGS

FIG. 1 is a cross-sectional view showing the cambium of a material plant (ginkgo tree).
FIG. 2 is a set of photograph showing the induction and isolation of stem cells according to the present invention, in which FIG. 2A shows that stem cells (arrow) have a morphology significantly different from phloem cells (asterisk), and FIG. 2B is a photograph of isolated stem cells after 3 weeks of culture.
FIG. 3 is a set of photographs showing a solid culture of stem cells according to the present invention (FIG. 3A) and a solid culture of a callus derived from the bark explant of a ginkgo tree (FIG. 3B).
FIG. 4 is a set of micrographs showing the degree of aggregation of cells in stem cells (FIG. 4A) during culture and in a callus (FIG. 4B) derived from the bark tissue of a ginkgo tree (100x magnification).
FIG. 5 is a set of micrographs showing stem cells according to the present invention (FIG. 5A) and the somatic cells of a ginkgo tree (FIG. 5B). Scale bar at the bottom of the left side of each photograph: 25 µm.
FIG. 6A is a micrograph showing a stem cell according to the present invention, observed after staining of the vacuole with neutral red, and FIG. 6B is a micrograph showing a callus derived from the bark tissue of a ginkgo tree. Scale bar at the bottom of the right side of each photograph: 25 µm.
FIG. 7 is a set of photographs showing mitochondria in stem cells according to the present invention (FIG. 7A) and in a callus derived from the bark tissue of a ginkgo tree (FIG. 7B).
FIG. 8 is a graphic diagram showing a growth curve of stem cells according to the present invention in a bioreactor.
FIG. 9 shows a comparison of the growth rate of stem cells according to the present invention with a callus derived from the bark tissue of a ginkgo tissue.
FIG. 10 is a set of photographs showing the result of culture for 10 days in a 3-liter air-lift bioreactor (FIG.10A), a 20-liter air-lift bioreactor (FIG. 10B) and a 250-liter air-lift bioreactor (FIG. 10C).
FIG. 11 is a graphic diagram showing the cell viability after cryspreservation of stem cells according to the present invention and a callus derived from the bark tissue of a ginkgo tree.
FIG. 12 is a graphic diagram showing the radical elimination abilities as function of the concentration of a stem cell extract (GG-E) according to the present invention and a stem cell extract treated with elicitors (GP-E).

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein is well known and commonly employed in the art.

The definition of main terms used in the detailed description of the invention is as follows.

As used herein, the vascular "cambium" refers to a lateral meristem located within vascular tissue of a plant and is located on a stem and a root. The thickening growth of plants occurs by the activity of the cambium, and as a result, giant plants having more than 11,000 years of the growth rings may exist. Embryologically, the vascular cambium originates from the procambium, and thus is the same meristem which has been gradually differentiated with meristematic continuity (Jae-Doo LEE and seven others, "Plant Morphology", Academy Book Publishing Co, Chapter 10, 1993). In the present invention, the term "cambium" is meant to include a procambium. Such cambium and procambium are primary meristem, and thus in the present invention, it is obvious that the use of such cambium and procambium provides the same effect.

As used herein, the term "stem cell" refers to an innately undifferentiated cell which has not undergone a dedifferentiation process and is thus genetically highly stable.

As used herein, the term "lysate" refers to a cell lysate obtained by disrupting cells through a chemical method, for example, using a detergent, or a physical method. The term "extract" of a cell line refers to a substance obtained by dissolving cells in a solvent and being isolated, and the extract can be concentrated through distillation or evaporation. Also, the term "culture medium" of a cell line refers to a cell medium solution from which cells have been removed after culturing the cells. Additionally, the term "culture" as used herein refers to a substance including a culture medium and/or a cultured cell line, wherein the cultured cell is meant to include all cell which differentiate under culture conditions or which have improved ability to produce and/or secrete useful substances.

As used herein, the term "innately undifferentiated" means that cells are not present in an undifferentiated state through a dedifferentiation process, but are originally maintained in a pre-differentiated state.

As used herein, the term "callus" refers to cells or a cell colony which has not differentiated through a dedifferentiation process (PNAS, 99(25):15843, 2002).

In one aspect, the present invention is directed to a stem cell derived from a cambium of family *Ginkgoaceae,* in which the stem cell is an innately undifferentiated cell without going through dedifferentiation.

When leaf, stem or root portions which are already differentiated tissues are used, they undergo a dedifferentiation process in which they are rejuvenated to their undifferentiated state in order to form a callus. During the dedifferentiation process, a mutation in somatic cells appears to cause cell unstability. Accordingly, the present inventors have conducted studies on a plant cell system showing little or no somaclonal variation, and as a result, have found that, when a cell line is derived specifically only from the meristem cambium, the ability of the metistem itself vigorously to divide can be used, and thus a cell line, which has no somaclonal variation and is genetically highly stable and physiologically uniform, can be derived without undergoing a dedifferentiation process. Based on this finding, a cambium-derived stem cell was isolated.

A stem cell derived from the cambium of the family *Ginkgoaceae* may have at least one of the following characteristics: (a) including a greater number of single cells or including smaller-sized cell aggregates in suspension culture than a dedifferentiated callus of the family *Ginkgoaceae*; (b) having a large number of vacuoles morphologically; (c) having mitochondria having increased activity compared to the dedifferentiated callus of the family *Ginkgoaceae*; (d) being capable of growing faster and longer than the dedifferentiated callus of the family *Ginkgoaceae*; and (e) having lower sensitivity to shear stress in a bioreactor than the dedifferentiated callus of the family *Ginkgoaceae.*

As used herein, the phrase "having a large number of vacuoles" means having a number of vacuoles which is at least two times greater than the number of vacuoles in the dedifferentiated callus of a plant of the family *Ginkgoaceae.* In addition, the stem cells according to the present invention have smaller vacuoles than the dedifferentiated callus of a plant of the family *Ginkgoaceae.* Furthermore, the phrase "having a number of developed mitochondria" as used herein means having a number of mitochondria moving actively under a microscope, which is at least two times greater than the number of mitochondria in the dedifferentiated callus of a plant of the family *Ginkgoaceae.*

In the present invention, the stem cells may have at least two of the above characteristics (a) to (e), preferably at least three of the above characteristics (a) to (e), and more preferably at least four of the above characteristics (a) to (e). In addition, the stem cells according to the present invention may also have all of the above characteristics (a) to (e).

In addition, the present inventors have found that stem cells derived from the cambium of the family *Ginkgoaceae* have the capacity to differentiate into tracheary elements. The cells derived from the cambium of the family *Ginkgoaceae* according to the present invention have self-renewal ability and pluripotency which are the characteristics of plant stem cells, indicating that the cells according to the present invention are stem cells.

The stem cell according to the present invention can be obtained by an isolation method comprising the steps of: a) obtaining a tissue comprising a cambium from a plant of the family *Ginkgoaceae*; (b) culturing the obtained cambium-comprising tissue in a medium; and (c) isolating a cell from the cambium, thereby obtaining a stem cell derived from the cambium. Step (b) of the method according to the present invention may be performed by culturing the cambium-comprising tissue to induce a cultured cambium proliferated from the cambium, and step (c) of the method may be performed by isolating the cultured cambium to obtain a cambium-derived stem cell line. Step (b) of the method may be performed by culturing the cambium-containing tissue in a medium containing auxin, in which auxin in the medium may be NAA (α-naphtalene acetic acid) or IAA (indole-3-acetic acid). Such auxin may be contained at a concentration of 1-5 mg/ℓ in the medium. Also, step (c) of the method may be performed by isolating the cultured cambium from an amorphous callus layer that proliferated from tissues other than the cambium, thereby obtaining the cambium-derived stem cell.

In one Example of the present invention, stem cells derived from the cambium of a ginkgo tree were isolated from a ginkgo tree, a member of the genus *Ginkgo* of the family *Ginkgoaceae.* Thus, the stem cells according to the present invention are preferably stem cells derived from the cambium of the genus *Ginkgo,* and more preferably stem cells derived from the cambium of a ginkgo tree.

Also, in the present invention, the stem cells derived from the cambium of a ginkgo tree, were found to have antioxidant effects.

In another aspect, the present invention is directed to an antioxidant composition containing any one or more of said stem cell derived from the cambium of the family *Ginkgoaceae,* an extract thereof, a lysate thereof, and a culture thereof.

In the present invention, the culture of the stem cell is preferably obtained by additionally culturing the stem cell in a medium, which, as elicitors, contains 3-5 wt% of raw sugar or sugar, and/or any one or more of methyl jasmonate, chitosan, phenylalanin, benzoic acid, ABA, salicylic acid and sodium acetate. Herein, the medium preferably contains 3-5 wt% of raw sugar or sugar, and at least one substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

Also, in the present invention, it is possible to use a culture medium obtained by treating the stem cell with elicitors, including UV radiation, heat, ethylene, an antifungal agent, an antibiotic, heavy metal salt and high-concentration salt, and applying physical and chemical stresses thereto.

In the present invention, the extract is preferably obtained using a solvent selected from the group consisting of distilled water, alcohol such as ethanol, acetone, DMSO (dimethyl sulfoxide), and mixed solvents thereof.

In one Examples of the present invention, it was found that an extract of the stem cells according to the present invention and an extract of a culture thereof had the ability to eliminate radicals caused by the oxidant DPPH. This radical elimination ability of the extracts of the stem cell and the culture thereof was equal or superior to that of BHT which has been widely used as a synthetic antioxidant. Thus, the extract of stem cell according to the present invention will be useful as a very effective antioxidant composition.

Thus, even though in the present invention, there is no specific example showing that a composition containing the stem cells or a lysate thereof shows antioxidant effects, it will be obvious to those skilled in the art that the composition containing the stem cells according to the present invention or a lysate thereof can also show antioxidant activity to inhibit oxidation, because the extracts of the stem cells and the culture thereof were found to have antioxidant activity as described above.

The antioxidant composition which contains, as an active ingredient, any one or more of the stem cell according to the present invention, an extract thereof, a lysate thereof and a culture thereof, may be provided as a pharmaceutical composition additionally containing at least one pharmaceutically acceptable carrier, excipient or diluent. The stem cell line or the like may be contained in a pharmaceutical composition in a pharmaceutically effective amount depending on disease and its severity, the patient's age, weight, health condition and sex, the route of administration and the period of treatment.

As used herein, the term "pharmaceutically acceptable composition" refers to a composition that is physiologically acceptable and does not cause gastric disorder, allergic reactions such as gastrointestinal disorder or vertigo, or similar reactions, when administered to humans. Examples of said carrier, excipient or diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, magnesium stearate and mineral oils.

The pharmaceutical composition of the present invention may additionally contain fillers, anti-aggregating agents, lubricants, wetting agents, perfumes, emulsifiers and preservatives. Also, the pharmaceutical composition of the present invention may be formulated using a method well known in the art, such that it can provide the rapid, sustained or delayed release of the active ingredient after administration to mammals. The pharmaceutical composition of the present invention may be in the form of powders, granules, tablets, emulsions, syrups, aerosols, soft or hard gelatin capsules, sterile injection solutions, sterile powders, etc.

In still another aspect, the present invention is directed to an antioxidant functional food containing, as active ingredients, any one or more of said stem cell derived from the cambium of the family *Ginkgoaceae,* an extract thereof, a lysate thereof, and a culture thereof.

As used herein, the term "functional food" refers to a food, the functionality of which has been improved by adding thereto any one or more of the stem cell according to the present invention, a lysate thereof, an extract thereof, or a culture thereof.

### EXAMPLES

Hereinafter, the present invention will be described in further detail with reference to examples. It will be obvious to a person having ordinary skill in the art that these examples are illustrative purposes only and are not to be construed to limit the scope of the present invention.

In the following Examples, the antioxidant effects of an extract of stem cells derived from the cambiums of a ginkgo tree and a culture thereof were confirmed. However, it will be obvious to those skilled in the art that the use of the stem cell itself or a lysate thereof can show the same results as those of the extract or culture of the stem cell.

### Example 1: Preparation of stem cells derived from cambium of plant of family Ginkgoaceae.

### 1-1: Preparation of plant material

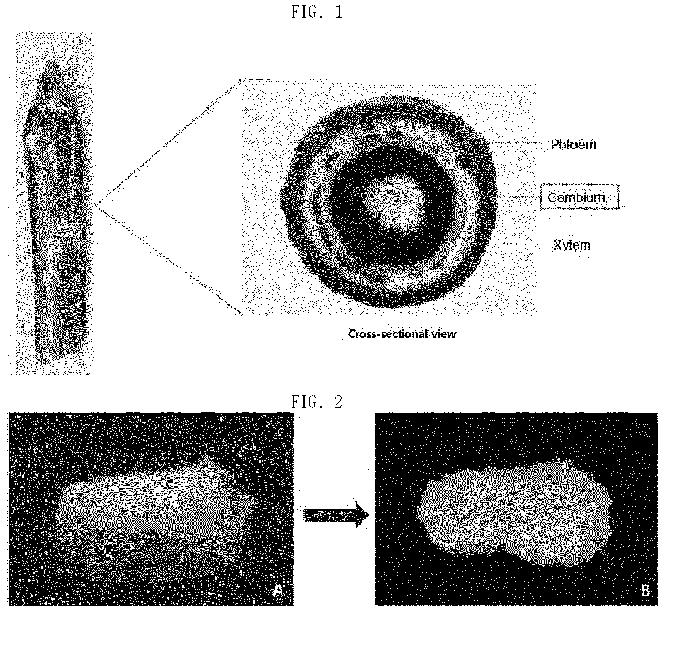

A ginkgo tree (*Ginkgo biloba*), a member of the genus *Ginkgo* of the family *Ginkgoaceae* was obtained from Buyeo-gun of Chungcheongnam-do, Korea, and stained with phloroglucinol as shown FIG. 1. As a result, the cambium could be observed between the xylem and the phloem. In order to obtain the cambium-derived stem cells from the ginkgo tree, stems from the ginkgo tree were collected, and then immediately settled in 100 mg/L of the antioxidant L-ascorbic acid (DUCHEFA, The Netherlands) solution. Then, they were transported and stored. Meanwhile, in order to obtain the same stem cells from the procambium of the ginkgo tree, twigs in place of stems were collected.

Then, the plant was pretreated with a mixed solution of 1% benomyl (Dongbu Hannong Chemical, Korea), 1% daconil (Dongbu Hannong Chemical, Korea), 1% streptomycin sulphate (DUCHEFA, The Netherlands) and 0.1% cefotaxime sodium (DUCHEFA, The Netherlands) for 24 hour, and then washed with tap water for 30 minutes to remove phenolic compounds and the remaining chemicals. Next, the plant was surface-sterilized in 70% ethanol (DC Chemical, Korea) for 1 min, 30% hydrogen peroxide (LG Chemical, Korea) for 15 min, 1% CLOROX solution for 15 min and 3% CLOROX solution for 5 min, and then washed 3-4 times with water.

### 1-2: Preparation of cambium-containing explant from stem and separation of tissue

The xylem of the stem (twig when using the procambium), sterilized in Example 1-1, was held with tweezers, and in this state, the bark tissue containing the cambium was peeled off from the xylem by pulling it. The peeled tissue was composed of an explant including cambium, phloem, cortex and epidermis.

### 1-3: Induction of ginkgo cambium-derived stem cells

The cambium-containing explant prepared in Example 1-2 above was planted and cultured in the stem cell induction medium (medium 1) shown in Table 1 below.

**[Table 1]**

| Cell line induction medium (medium 1) | | |
|---|---|---|
| | Composition | Contents(mg/L) |
| Inorganic salts | KNO₃ | 2500 |
| | (NH₄)₂SO₄ | 134 |
| | MgSO₄·7H₂O | 121.56 |
| | MnSO₄·4H₂O | 10 |
| | ZnSO₄·7H₂O | 2 |
| | CuSO₄·5H₂O | 0.025 |
| | CaCl₂·2H₂O | 113.23 |
| | KI | 0.75 |
| | CoCl₂·6H₂O | 0.025 |
| | NaH₂PO_{4·}H₂O | 130.44 |
| | H₃BO₃ | 3 |
| | Na₂MoO₄·2H₂O | 0.25 |
| | FeNaEDTA | 36.7 |
| Vitamin | Myo-inositol | 200 |
| | Thiamine-HCl | 20 |
| | Nicotinic acid | 2 |
| | Pyridoxine-HCl | 2 |
| | L-ascorbic acid | 50 |
| | Citric acid | 75 |
| Amino acid | L-aspartic acid | 133 |
| | L-arginine | 175 |
| | Glycine | 75 |
| | Proline | 115 |
| Hormone | α-Naphtalene acetic acid | 2 |
| Sucrose | | 10,000 |
| Activated charcoal | | 100 |
| Gelrite | | 2,000 |

The growth regulator auxin such as NAA or IAA may be added to the medium at a concentration of 1-5mg/L, and preferably 2 mg/L. The culture was carried out in a dark room controlled at 25+1 °C.

At 4-7 days of initial culture, the division of cells from the cambium was visually observed, and after 15 days of culture, an amorphous callus formed by dedifferentiation started to be induced from the layer composed of phloem, cortex and epidermis. After 30 days of culture, the cultured cambium started to be separated from the phloem-containing layer, that is, an amorphous callus layer (FIG. 2A: stem cell (arrow); phloem cell (asterisk). After the tissue has been naturally completely separated into the two layers, the layers were separately cultured in different Petri dishes. After the tissue has been isolated, the white and soft portion thereof having good growth rate was subcultured in the same fresh medium as induction medium at an interval of 14 days. FIG. 2B is a photograph showing the isolated, cambium-derived stem cells after 3 weeks of cultures.

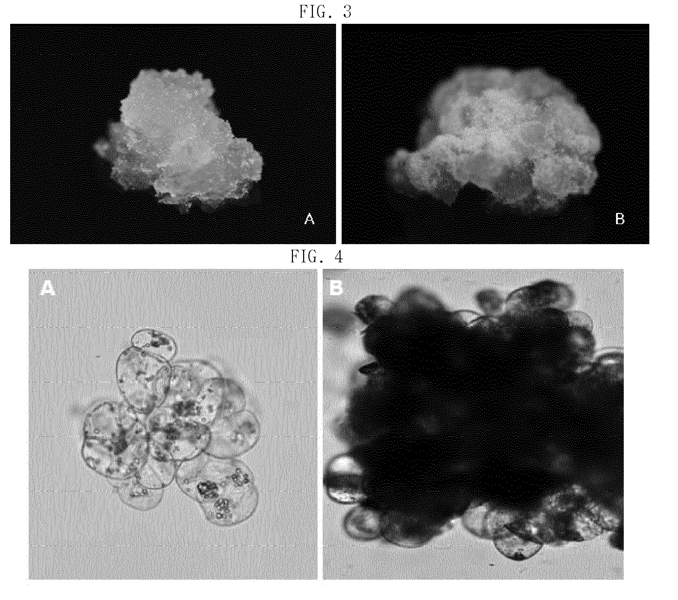

For comparison, the bark explant of the ginkgo tree were sterilized, and then cultured in the medium of Table 1. As a result, as can be seen in FIG. 3B, the bark explant formed a callus by dedifferentiation. The callus derived from the bark explant had an irregular shape due to the difference in division rate between different cells, showed unstable growth rate and readily turned brown. The brown and aggregated calluses showed slow growth due to phenolic compounds secreted by themselves, and eventually necrotized. In other words, after 6 months, the calli derived from the bark were difficult to maintain and culture. On the contrary, as shown in FIG. 3A, the stem cells derived from the cambium of the ginkgo tree were stably maintained without variations in their growth rates, growth patterns and aggregation degrees when they were cultured for a long period, suggesting that the large scale cell culture would be possible.

### Example 2: Proliferation of stem cells derived from cambium of the genus Ginkgo and observation of characteristics of the stem cells

The cambium-derived stem cells isolated in Example 1 above were placed in a flask containing the liquid medium shown in Table 2 below. Then, the stem cells in the flask were cultured in a rotating shaker under dark conditions at 100 rpm at 25 ±1 °C. The interval of subculture was set at 2 weeks, such that the cultured cells could always be maintained in high activity in the exponential growth phase.

**[Table 2]**

| | | |
|---|---|---|
| Suspension medium (medium 2) | | |

| | Composition | Contents(mg/L) |
|---|---|---|
| Inorganic salts | Ca(NO₃)₂ | 471.26 |
| | NH₄NO₃ | 400 |
| | MgSO₄· 7H₂O | 180.54 |
| | MnSO₄· 4H₂O | 22.3 |
| | ZnSO₄· 7H₂O | 8.6 |
| | CuSO₄· 5H₂O | 0.25 |
| | CaCl₂· 2H₂O | 72.5 |
| | K₂SO₄ | 990 |
| | Na₂MoO_{4·} 2H₂O | 0.25 |
| | H₃BO₃ | 6.2 |
| | KH₂PO₄ | 170 |
| | FeNaEDTA | 36.7 |
| Vitamin | Myo-inositol | 200 |
| | Thiamine-HCl | 20 |
| | Nicotinic acid | 2 |
| | Pyridoxine-HCl | 2 |
| | L-ascorbic acid | 50 |
| | Citric acid | 75 |
| Amino acid | L-aspartic acid | 133 |
| | L-arginine | 175 |
| | Glycine | 75 |
| | Proline | 115 |
| Hormone | α-Naphtalene acetic acid | 2 |
| Sucrose | | 30,000 |

The degree of aggregation of the cells was observed with biological microscope CX31 (Olympus, Japan). As a result, as shown in FIG. 4A, it was observed that the stem cells according to the present invention included a large number of single cells during suspension culture, and some of the cells were present as small-sized cell aggregates. However, in a control, the somatic cells (callus derived from the bark tissue) of the ginkgo tree were aggregated as shown in FIG. 4B.

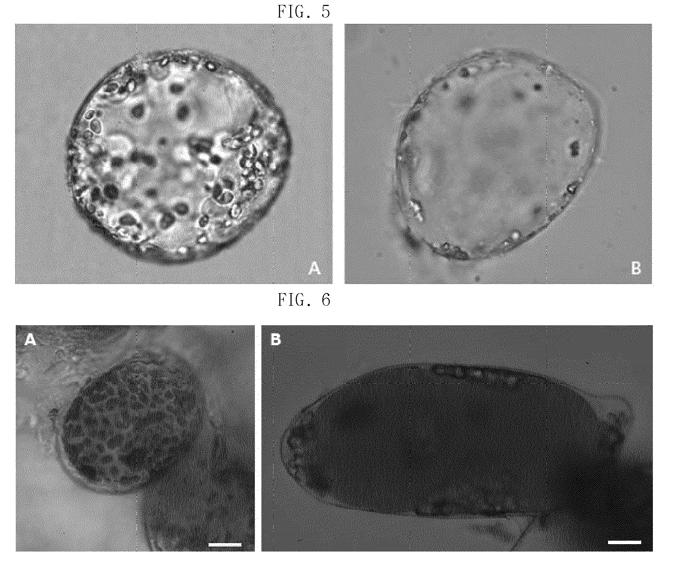

Meanwhile, as shown in FIG. 5A, the stem cells according to the present invention were morphologically characterized by a large number of vacuoles. This characteristic appears in undifferentiated cells in plants by factors such as pressure, suggesting that the stem cells according to the present invention are in an undifferentiated state. On the contrary, as shown in FIG. 5B, this characteristic could not be observed in the somatic cells (callus derived from the bark tissue) of the ginkgo tree. To demonstrate this observation, the vacuoles were stained with neutral red. As a result, as shown in FIG. 6A, the stem cell according to the present invention had a number of vacuoles, and as shown in FIG. 6B, the somatic cell (callus derived from the bark tissue) of the ginkgo tree had one large central vacuole.

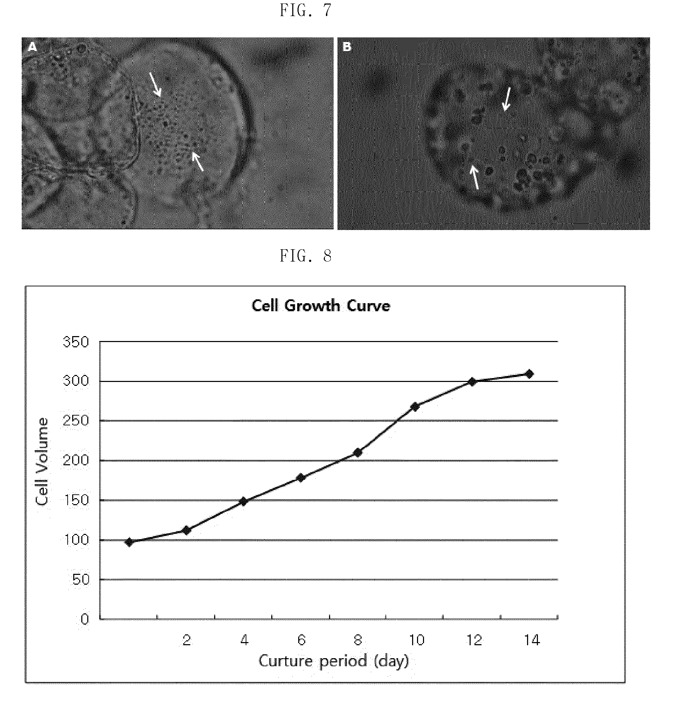

Meanwhile, the stem cells according to the present invention were observed with optical microscope BX41TF. As a result, a large number of mitochondria having a very high activity in terms of movement could be observed in the stem cell. FIG. 7A shows that the stem cell according to the present invention has a large number of mitochondria. In FIG. 7A, the arrow indicates mitochondria. On the contrary, as can be seen in FIG. 7B, this characteristic could not be seen in the somatic cell (callus derived from the bark tissue) of the ginkgo tree.

Meanwhile, in order to measure the growth rate of cells at various time points of culture, the growth rate of cells was measured using an airlift bioreactor (Samsung Science Co., Ltd., Korea) having an internal volume of 3 L. As a result, as shown in FIG. 8, growth rates of 2 fold at 1 week of culture and 3 fold at 2 weeks of culture could be observed. The culture was carried out in the liquid medium of Table 2 under dark conditions at 25+1 °C. In addition, the callus derived from the bark tissue of the ginkgo tree and the ginkgo cambium-derived stem cells according to the present invention were cultured under the above conditions, and the culture results were compared between the callus and the stem cell.

**[Table 3]**

| | | |
|---|---|---|
| Results of measurement of growth rate and GI | | |

| Cell lines | Growth rate (folds) | GI |
|---|---|---|
| Stem Cell | 3.1 | 2.27 |
| Callus | 2.3 | 1.3 |

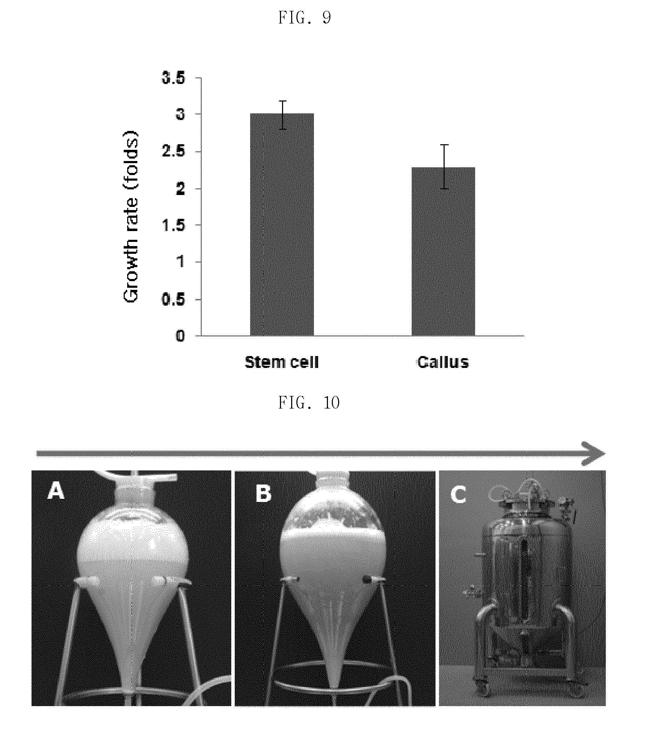

As a result, as shown in Table 3 above and FIG. 9, the callus derived from the bark tissue of the ginkgo tree showed a growth rate of 2.3 fold and a GI (growth index = (maximum DCW - initial DCW) / initial DCW) of only 1.3, whereas the cambium-derived stem cells according to the present invention showed a growth rate of 3.1 fold and a GI of 2.27, which were higher than those of the callus derived from the bark tissue of the ginkgo tree. In the case of conventional reactors, cell viability rapidly decreases due to growth ring production in the reactor, plant cell aggregation during culture, and the sensitivity of hard cell walls to shear stress. However, the cambium-derived stem cells formed a very small growth ring area in the bioreactor during culture, and the ring formed on the internal wall thereof was simply eliminated when a simple stimulus was applied to the bioreactor. Also, it was shown that the cambium-derived stem cells had low aggregation and contained a large number of vacuoles, and thus had low sensitivity to shear stress, such that cell viability did not decrease.

**[Table 4]**

| | | |
|---|---|---|
| Large-scale culture process | | |

| | Bioreactors | Max. dry cell weight (g/L) |
|---|---|---|
| A | 3L air-lift bioreactor | 11.3 |
| B | 20L air-lift bioreactor | 10.1 |
| C | 250L air-lift bioreactor | 11.7 |

In addition, as can be seen in Table 4 and FIG. 10, when the stem cells were cultured in each of the 3-L air-lift bioreactor, the 20-L air-lift bioreactor and the 250-L air-lift bioreactor for 10 days, it was shown that the cells grew even in the 250-L bioreactor and that the amount of dry cells per liter in the 250-L bioreactor increased rather than decreased.

In other words, it was seen that the cambium-derived stem cells according to the present invention had low sensitivity to shear stress in the bioreactor for scale-up culture, and thus could be produced rapidly in large amounts in the bioreactor. Accordingly, it could be seen that the cambium-derived stem cells according to the present invention had lower sensitivity to shear stress than cell derived from the dedifferentiated callus of the ginkgo tree.

Meanwhile, the callus derived from the bark tissue of the ginkgo tree and the inventive stem cells derived from the cambium of the ginkgo tree were cryopreserved. A used suspension culture was culture incubated for 6-8 days, and a cryopreservative was a medium containing 0.5M glycerol (DUCHEFA, The Netherlands), 0.5M DMSO (DUCHEFA, The Netherlands) and 1M sucrose (DUCHEFA, The Netherlands) and was transferred into a 5ml cryovial (Duran, USA). The amount of cells inoculated into the cryopreservative was 200 mg/ml. The suspended cells treated with the cryopreservative were frozen by maintaining them in a freezer for 30 minutes, storing them in a deep freezer for 3 hours, and then soaking them in liquid nitrogen.

Then, for thawing, the cultured cells maintained in liquid nitrogen for 20 minutes or more were taken out, placed in a constant-temperature water bath at 40 °C and thawed for 1-2 minutes. For cell regrowth, the cell suspension was filtrated through a sterilized funnel and filter paper. The filtrated cells were applied on a solid growth medium including filter paper, and they were stabilized at room temperature for 30 minutes, and then transferred to a fresh solid growth medium.

**[Table 5]**

| | | | | |
|---|---|---|---|---|
| Results of measurement of cell viability during cryopreservation | | | | |
| Stem cell 1 | 33/212 | 43/218 | 45/211 | 18.87% |
| | 15.60% | 19.70% | 21.30% | |
| Callus | 5/175 | 4/124 | 4/158 | 2.87% |
| | 2.90% | 3.20% | 2.50% | |

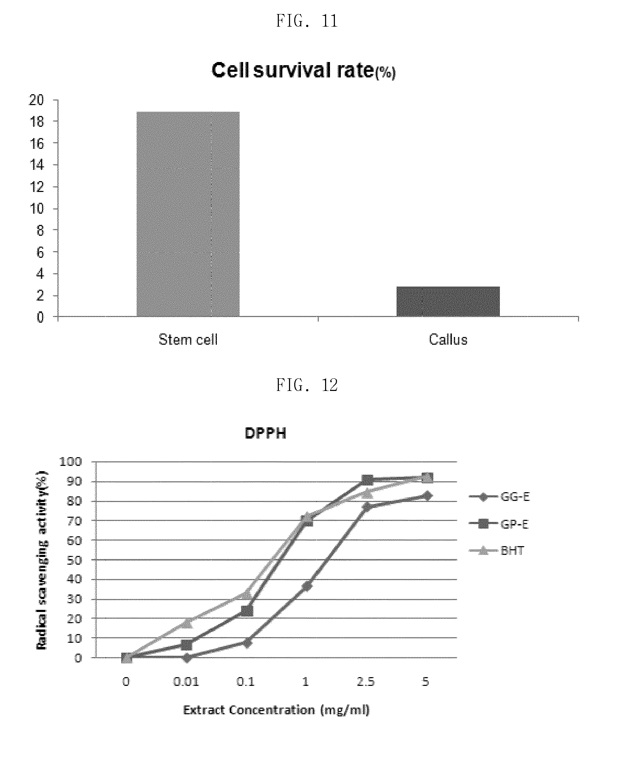

As a result, as can be seen in FIG. 11 and Table 5,the callus derived from the bark tissue of the ginkgo tree showed a very low cell viability compared to the stem cells derived from the cambium of the ginkgo tree according to the present invention.

In addition, the characteristics of plant stem cells include self-renewal ability and pluripotency. Thus, in order to induce the ginkgo cambium-derived stem cells to differentiate into tracheary elements, the stem cells were cultured in MS medium containing a cell growth regulator at 25+1 °C under dark conditions. As a result, it could be seen that the cambium-derived stem cells differentiated into tracheary elements.

### Example 3: Treatment with elicitor and preparation of extract of stem cells derived from cambium of ginkgo tree

### 3-1: Treatment with elicitor

The stem cells, which have been suspension-cultured for 14 days as described in Example 2, were divided into the following two groups for experiments: (1) the cell line (growth phase) suspension-cultured for 14 days; and (2) a cell line (elicitation phase), obtained by culturing the 14-day suspension-cultured cell line in a medium (containing 3-5 wt% (g/L) raw sugar and 100 µM methyl jasmonate in sterile water) in a dark condition for 14 days.

### 3-2: Preparation of extract

Each of the two cell lines from which the culture medium has been removed was freeze-dried. Then, 2 g of each freeze-dried cell line (dry) was stirred in 50 ml of 80% ethanol with stirring at 15 °C for 48 hours for dissolution purpose. After completion of the dissolution, each of the cell solutions was centrifuged at 3,000 rpm for 20 minutes, and each of the supernatants was freeze-dried. Each of the resulting extract powders was dissolved in PBS, thereby obtaining an ethanol extract of each of the two cell lines.

### Example 4: Examination of antioxidant effect of stem cells derived from cambium of ginkgo tree

In order to examine the antioxidant effect of the stem cells according to the present invention, each of the ethanol extracts prepared in Example 3-2 was oxidized by DPPH, and then the radical elimination ability of each extract was measured.

Specifically, 20 µℓ of each ethanol extract and 180 µℓ of 100 µM DPPH solution were added to each well of a 96-well plate and allowed to react with each other at room temperature for 20 minutes. Then, the absorbance of each well at 520 nm was measured. BHT which is widely used as a synthetic antioxidant, was used as a control and radical scavenging ability was shown as percentages.

As a result, as can be seen in FIG. 12, the ethanol extract (GG-E: growth phase ethanol extract) obtained from the 14-day suspension-cultured cell line treated with no elicitor showed an increase in antioxidant activity in a concentration-dependent manner and showed an antioxidant activity similar to BHT (which has been used as a synthetic antioxidant) at a concentration of 2.5 mg/ml, suggesting that it has excellent antioxidant activity. Also, the ethanol extract (GP-E: elicitation phase ethanol extract) obtained from the cell line treated with raw sugar and methyl jasmonate as elicitors showed an increase in antioxidant activity in a concentration-dependent manner and showed an antioxidant activity higher than BHT at a concentration of 2.5 mg/ml. Such test results suggest that the cambium-derived stem cells according to the present invention have an excellent antioxidant effect.

### Preparation Example 1: Preparation of pharmaceutical formulations

### Formulation 1-1: Preparation of tablet

100 mg of the stem cell extract prepared in Example 3 was mixed with 100 mg of maize starch, 100 mg of lactose and 2 mg of magnesium stearate, and the mixture was compressed into a tablet according to a conventional tableting method.

### Formulation 1-2: Preparation of capsule formulation

500 mg of the stem cell extract prepared in Example 3 was filled in a soft gelatin capsule to prepare a capsule formulation.

### Formulation 1-3: Preparation of syrup formulation

1 g of the stem cell prepared in Example 1 was mixed with 10 g of isomerized sugar, 5 g of mannitol and a suitable amount of purified water, and the mixture was prepared into 100 ml of a syrup formulation according to a conventional method.

### Formulation 1-4: Preparation of injection solution

200 mg of the stem cell extract prepared in Example 3 was heated and dissolved in 200 mg of physiological saline containing polyoxyethylene hydrogenated castor oil, thus preparing an injection solution containing the stem cell extract at a concentration of 0.1%.

### Preparation Example 2: Preparation of functional foods

### Preparation Example 2-1: Preparation of functional beverage

200 mg of the stem cell prepared in Example 1 was dissolved in 96 ml of water, and then 500 mg of vitamin C as a supplement, 1 g of each of citric acid and oligosaccharide as flavor enhancers and 0.05 g of sodium benzoate as a preservative were added thereto. Then, purified water was added thereto, thus preparing 100 ml of a functional beverage.

### Preparation Example 2-2: Preparation of functional beverage

200 mg of the stem cell extract prepared in Example 3 was dissolved in 96 ml of water, and then 500 mg of vitamin C as a supplement, 1 g of each of citric acid and oligosaccharide as flavor enhancers and 0.05 g of sodium benzoate as a preservative were added thereto. Then, purified water was added thereto, thus preparing 100 ml of a functional beverage.

### INDUSTRIAL APPLICABILITY

As described above, the inventive stem cell line derived from the cambium of a plant of the family *Ginkgoaceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell line has an antioxidant effect which is similar to or higher than that of an existing antioxidant, as demonstrated by measuring the ability to eliminate radicals caused by oxidants. Thus, the disclosed stem cell line is useful as an effective antioxidant composition.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A stem cell derived from a cambium of family *Ginkgoaceae,* which is an innately undifferentiated cell without going through dedifferentiation.

2. The stem cell according to claim 1, wherein the stem cell additionally comprises at least one of the following characteristics:
(a) including a greater number of single cells or including smaller-sized cell aggregates in suspension culture than a dedifferentiated callus of the family *Ginkgoaceae*;
(b) having a large number of vacuoles morphologically;
(c) having mitochondria having increased activity compared to the dedifferentiated callus of the family *Ginkgoaceae*;
(d) being capable of growing faster and longer than the dedifferentiated callus of the family *Ginkgoaceae*; and
(e) having lower sensitivity to shear stress in a bioreactor than the dedifferentiated callus of the family *Ginkgoaceae.*

3. The stem cell according to claim 2, wherein the stem cell comprises two or more characteristics selected from the group consisting of the characteristics (a) to (e).

4. The stem cell according to claim 2, wherein the stem cell comprises three or more characteristics selected from the group consisting of the characteristics (a) to (e).

5. The stem cell according to claim 2, wherein the stem cell comprises four or more characteristics selected from the group consisting of the characteristics (a) to (e).

6. The stem cell according to claim 2, wherein the stem cell comprises the characteristics (a) to (e).

7. The stem cell according to claim 1, wherein stem cell is obtained by an isolation method comprising the steps of:
(a) obtaining a tissue comprising a cambium from a plant of the family *Ginkgoaceae*;
(b) culturing the obtained cambium-comprising tissue in a medium; and
(c) isolating a cell from the cambium, thereby obtaining a stem cell derived from the cambium.

8. The stem cell according to any one claim among claims 1 to 7, wherein the plant of the family *Ginkgoaceae* is genus *Ginkgo.*

9. The stem cell according to any one claim among claims 1 to 7, wherein the plant of the family *Ginkgoaceae* is a ginkgo tree (*Ginkgo Biloba).*

10. A method of isolating a stem cell derived from a cambium of family *Ginkgoaceae,* the method comprising the steps of:
(a) obtaining a tissue comprising a cambium from a plant of the family *Ginkgoaceae*;
(b) culturing the obtained cambium-comprising tissue in a medium; and
(c) isolating a cell from the cambium, thereby obtaining a stem cell derived from the cambium.

11. The method according to claim 10, wherein the medium of the step (b) comprises auxin.

12. The method according to claim 11, wherein the medium comprises 1∼5mg/L of auxin.

13. The method according to claim 10, wherein the step (c) comprises isolating cells from the cambium after separating the cultured cambium from an amorphous callus layer derived from tissues other than the cambium, thereby obtaining the stem cell derived from the cambium.

14. The method according to claim 10, wherein the plant of the family *Ginkgoaceae* is genus *Ginkgo.*

15. The method according to claim 10, wherein the plant of the family *Ginkgoaceae* is a ginkgo tree.

16. An antioxidant composition containing any one or more of the stem cell derived from the cambium of the family *Ginkgoaceae* of any one claim among claims 1 to 7; an extract thereof; a lysate thereof; and a culture thereof.

17. The antioxidant composition according to claim 16, wherein the culture is obtained by additionally culturing the stem cell in a medium containing 3-5 wt% of raw sugar or sugar; or one or more substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

18. The antioxidant composition according to claim 16, wherein the plant of the family *Ginkgoaceae* is genus *Gingko.*

19. The antioxidant composition according to claim 16, wherein the plant of the family *Ginkgoaceae* is a ginkgo tree.

20. An antioxidant functional food, which contains any one or more of the stem cell derived from the cambium of the family *Ginkgoaceae* of any one claim among claims 1 to 7; an extract thereof; a lysate thereof; and a culture thereof.

21. The antioxidant functional food according to claim 20, wherein the culture is obtained by additionally culturing the stem cell in a medium containing 3-5 wt% of raw sugar or sugar; or one or more substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

22. The antioxidant functional food according to claim 20, wherein the plant of the family *Ginkgoaceae* is genus *Ginkgo.*

23. The antioxidant functional food according to claim 20, wherein the plant of the family *Ginkgoaceae* is a ginkgo tree.
